# EUROPÄISCHE PATENTSCHRIFT

(11) **EP 0 355 536 B1**
(45) Veröffentlichungstag und Bekanntmachung des Hinweises auf die Patenterteilung: **13.11.1996**
(21) Anmeldenummer: 89114487.5
(22) Anmeldetag: 05.08.1989
(51) Int. Cl.: C08J 5/18, A61L 15/16, A61L 25/00

(54) **Flexibler, hydrophiler Gelfilm, Verfahren zu seiner Herstellung und Verwendung**
Flexible hydrophilic gel film, process for its preparation and use
Film de gel flexible hydrophile, procédé pour sa fabrication et utilisation

(30) Priorität: 13.08.1988 DE 3827561
(43) Veröffentlichungstag der Anmeldung: 28.02.1990
(73) Patentinhaber: LTS LOHMANN Therapie-Systeme GmbH, 56567 Neuwied (DE)
(72) Erfinder: Roreger, Michael, D-5450 Neuwied 12 (DE); Herrmann, Fritz, Dr., D-5450 Neuwied 12 (DE); Hoffmann, Hans-Rainer, Dr., D-5450 Neuwied 22 (DE); List, Harald, Dr., D-5450 Neuwied 1 (DE)
(74) Vertreter: Flaccus, Rolf-Dieter, Dr.

(56) Entgegenhaltungen:
- EP-A- 0 089 152
- EP-A- 0 138 385
- DE-A- 1 669 604
- GB-A- 2 036 042
- DATABASE WPIL, Derwent Publications Ltd, London, GB; & JP-A-01156341

## Beschreibung

Die Erfindung betrifft ein Verfahren zur Herstellung eines flexiblen, hydrophilen, in Wasser quellbaren, nicht löslichen Gelfilms, enthaltend
- 0,5 bis 30 Gew.-% mindestens eines wasserlöslichen bei neutralem pH anionaktiven Polymers,
- 0,5 bis 50 Gew.-% mindestens eines wasserlöslichen bei neutralem pH kationaktiven Polymers,
- 0,1 bis 20 Gew.-% mindestens eines Feuchthaltemittels,
- 0,1 bis 70 Gew.-% Wasser bzw. wässrige Lösemittel,
- 0 bis 75 Gew.-% wasserlösliche oder wasserdispergierbare Hilfsstoffe,
- 0 bis 50 Gew.-% Wirkstoff

Hydrophile Filme und filmförmige Hydrogele finden in den letzten Jahren auf den unterschiedlichsten Gebieten der Pharmazie und Medizin zunehmend mehr Verwendung, so z.B. als Wundauflagen, Überzüge für verschiedene Arzneiformen und Substrate, Wirkstoffträger für orale, bukkale, rektale, vaginale, dermale oder transdermale Applikation von Wirkstoffen, Implantate und künstliche Gefäße, um nur einige zu nennen (ausführliche Übersicht in: J.D. Andrade, Ed., Hydrogels for medical and related applications, 1976/N.A. Peppas, Ed., Hydrogels in Medicine and Pharmacy, Vol. I, II, III, 1987).

Das steigende Interesse für die Anwendung von Hydrogelen auf intakter und geschädigter Haut oder auf Schleimhäuten liegt vor allem darin begründet, daß Hydrogele meist eine deutlich bessere Verträglichkeit mit biologischen Oberflächen und Geweben aufweisen als weniger hydrophile Materialien, insbesondere wenn sie über einen längeren Zeitraum appliziert werden.

So haben wasserquellbare hydrophile Filme nach Applikation auf die Haut in der Regel ein geringeres Hautreizungspotential als Okklusivfilme oder -membranen, die allein aufgrund des Okklusiveffekts bei Langzeitapplikation Hautreizungen verursachen können. Zudem bietet die Herstellung aus wäßriger Lösung oder Dispersion gegenüber der Herstellung aus organischer Lösung hinsichtlich Toxikologie und Umweltbelastung deutliche Vorteile.

Hydrogele werden im allgemeinen unterteilt in hitzereversible und irreversible Gele. Unter irreversiblen Hydrogelen werden Gele verstanden, bei denen durch Vernetzung von Monomeren ein dreidimensionales stabiles Netzwerk aus Polymerketten mit kovalenten und physikalischen Bindungen aufgebaut wird. Diese Hydrogele können unter Wasseraufnahme quellen, sind in Wasser aber auch bei Erwärmen nicht mehr löslich.

Diese Hydrogele lassen sich aufgrund der Vernetzung sehr gut zur Herstellung stabiler Gelfilme verwenden. Die größte Bedeutung für Medizin und Pharmazie haben dabei Polymerfilme auf der Basis von verschiedenen Acrylsäure- und Methacrylsäuremonomeren erlangt. Diese Filme besitzen eine hohe Reißfestigkeit in gequollenem Zustand, allerdings ist das Quellvermögen begrenzt. Für bestimmte Verwendungszwecke können solche Polymerfilme durch Zugabe entsprechender Hilfsstoffe selbstklebend ausgerüstet werden, doch wird das Haftvermögen bei Kontakt mit Wasser oder durch Restwasser im Film stark vermindert.

Nachteil der irreversiblen Hydrogele ist vor allem, daß ihre Verwendbarkeit wegen der toxikologischen Eigenschaften der Monomere bzw. der eingesetzten Vernetzungsmittel insbesondere auf Schleimhäuten oder geschädigtem Gewebe häufig eingeschränkt ist.

Hitzereversible Hydrogele werden in der Regel hergestellt durch Auflösen hydrophiler Polymere in heißem Wasser und anschließendes Abkühlen, wobei beim Abkühlen unter den Gelierungspunkt die Gelbildung einsetzt. Diese Hydrogele haben eine geringere mechanische Stabilität, insbesondere wenn sie mit Wasser in Kontakt kommen. Moleküle werden aus dem Gel, wenn auch langsam, herausgelöst und die Reißfestigkeit nimmt mit zunehmender Quellung ab, wobei dieser Effekt z.B. bei der Anwendung auf der Haut durch die Erwärmung auf Körpertemperatur noch verstärkt wird. Vorteil ist, daß durch Auswahl geeigneter physiologisch unbedenklicher Polymere und Hilfsstoffe Hydrogele hergestellt werden könnnen, die auch zur Anwendung auf stark geschädigtem Gewebe oder in Körperhöhlen verwendet werden können. Allerdings werden bei diesen Hydrogelen Filme häufig erst nach der Applikation, z.B. nach Aufstreichen oder Aufsprühen, am Applikationsort selbst hergestellt, was beispielsweise bei wirkstoffhaltigen Hydrogelen eine genaue Wirkstoffdosierung unmöglich macht. Hinzu kommt, daß mit den verwendeten strukturbildenden Polymeren und Polymerkombinationen häufig nur ein bestimmter, sehr enger Anwendungsbereich abgedeckt werden kann.

Die EP-A-0 089 152 beschreibt die Herstellung eines flexiblen Gelfilmes auf einem anionischen Polymer und einem kationischen Polymer. Dabei wird aus einer wäßrigen Chitosanacetat-Lösung, die auch Glycerin enthält, zuerst ein Film gegossen, der nach dem Trocknen mit einer wäßrigen Ammoniumkeratinat-Lösung in Kontakt gebracht und dabei gequollen wird.

Aus der DE-A-1 669 604 ist es bekannt, ein anionaktives Polymer und ein kationaktives Polymer in einem wäßrigen Lösungsmittel, das genügend Säure oder Base oder ionische Salze enthält, in Lösung zu halten, so daß keine Reaktion der an sich unverträglichen Polymere eintritt. Der Zusatz wird entweder durch Verdampfen oder durch Neutralisation und Waschen entfernt. Aus diesem Dokument ist es weiter bekannt, ein Lösemittel zu verwenden, welches Natriumbromid enthält. Nach dem Gelieren durch Entfernen des Lösemittels wird restliches NaBr durch Waschen entfernt oder es kann, wenn poröse Gelprodukte gewünscht sind, auch direkt getrocknet werden. Dabei ist jedoch nicht auszuschließen, daß Reste von Bromid in dem hergestellten Gel rückständig vorhanden sind.

Ausgehend vom vorgenannten Stand der Technik liegt der Erfindung die Aufgabe zugrunde, einen hydrophilen, flexiblen Gelfilm herzustellen, der, ohne daß Vernetzungsmittel eingesetzt werden, bei hohem Wasseraufnahmevermögen in gequollenem Zustand eine mechanische Stabilität aufweist, die eine problemlose Handhabung ermöglicht, der aus Komponenten mit optimaler Haut- und Schleimhautverträglichkeit hergestellt ist und bei Bedarf mit Wirkstoff beladen werden kann.

Überraschenderweise wurde die Lösung der Aufgabe in einem Verfahren mit den im Hauptanspruch angegebenen Arbeitsschritten gefunden.
Weitere vorteilhafte Ausgestaltungen des Verfahrens sind entsprechend den Merkmalen der Ansprüche 2-16 vorgesehen. Es wird ein die Reaktion der Polymeren unterschiedlicher Ladung durch reversible Salzbildung verhindernder flüchtiger Zusatz basischen oder sauren Charakters verwendet, der eine Siedetemperatur zwischen 25 °C und 120 °C , vorzugsweise zwischen 40 °C und 80 °C aufweist.
Wasserlösliche, bei neutralem pH anionaktive Polymere können sein anionaktive Cellulosederivate, anionaktive Stärkederivate, anionaktive Galaktomannanderivate, anionaktive pflanzliche Polysaccharide, anionaktive Polyacrylate und Polymethacrylate. Anionaktive Cellulosederivate sind beispielsweise Natriumcarboxymethylcellulose, Celluloseacetatphthalat, Hydroxypropylmethylcellulosephthalat (HPMPC), Methylcellulosephthalat, Carboxymethylethylcellulose, Ethylcarboxyethylcellulose, Celluloseacetatsuccinat, Methylcellulosesuccinat, Methylcellulosephthalatsuccinat oder Ethylcellulosesuccinat. Anionaktive Stärkederivate sind bspw. Natriumcarboxymethylstärke oder Stärkeacetatphthalat, anionaktive Galaktomannane sind bspw. Galaktomannanphosphorsäureester (Meyprofilm^{R}, Fa. Meyhall), Carboxymethylguar (Meyprogum^{R}, Fa. Meyhall) und Carboxymethylhydroxypropylguar (Jaguar CMHP^{R}, Fa Meyhall), anionaktive pflanzliche Polysaccharide sind bspw. Pektine, Carrageenane, Alginate oder Xanthan (Keltrol^{R}), anionaktive Polyacrylate oder Polymethacrylate sind bspw. Copolymerisate auf Basis von Methacrylsäure und Methacrylsäuremethylestern (Eudragit L/S^{R}, Fa. Röhm-Pharma) oder Copolymere auf Basis von Acrylsäure, Acrylsäureestern und Methacrylsäureestern mit freien Carboxylgruppen (Aquakeep^{R}, Fa. Itoh/Carboset^{R}, Fa. Goodrich).

Wasserlösliche, bei neutralem pH kationaktive Polymere können sein Typ-A-Gelatine, kationaktive Galaktomannanderivate, wie bspw. Guarhydroxypropyltriammoniumchlorid (Jaguar C^{R}, Fa. Meyhall), sowie kationaktive Polyacrylate und Polymethacrylate, wie bspw. Copolymerisate von Dimethylaminoethylmethacrylat und neutralen Methacrylsäure (Eudragit E^{R}, Röhm-Pharma) oder Copolymerisate aus Acryl- und Methacrylsäurestern mit einem geringen Gehalt an quartären Ammoniumgruppen (Eudragit RL/RS^{R}, Röhm-Pharma). Als Feuchthaltemittel kann der Gel film Alkohole wie Glycerin, Propyllenglykol, Sorbit oder niedermolekulare Polyethylenglykole enthalten.

In der Fachliteratur findet man häufig den Hinweis, daß die Kombination bestimmter anionaktiver und kationaktiver Polymere in wäßriger Lösung nicht möglich ist, da es durch die gegensätzlichen Ladungen der Polymere zu Unverträglichkeiten und Klumpenbildung während des Lösungsvorgangs kommt, so z.B. bei der Kombination von Natriumcarboxymethylcellulose und Gelatine unterhalb ihres isoelektrischen Punkts, der bei Typ-A-Gelatine bei einem pH von etwa 8,5-9.0 liegt, oder bei der Kombination von anionaktiven, pflanzlichen Polysacchariden mit Typ-A-Gelatine (Literatur: 1) E. Doelker, "Water-swollen Cellulose Derivatives in Pharmacy", im: N.A. Peppas, Hydrogels in Medicine and Pharmacy, Vol. II, CRC press 1987, S. 122; 2) H.P. Fiedler, Lexikon der Hilfsstoffe für Pharmazie, Kosmetik und angrenzende Gebiete, Editio-Cantor Aulendorf 1981, S. 423).

Mit der vorliegenden Erfindung wurde ein Weg gefunden, diese Unverträglichkeitsreaktion zwischen anionaktiven und kationaktiven Polymeren während des Lösevorgangs zu vermeiden, indem man dem Lösemittel, das Wasser oder eine Mischung aus Wasser und mit Wasser mischbarem Lösemittel ist, einen die Reaktion von Polymeren unterschiedlicher Ladung verhindernden flüchtigen Zusatz, der eine Siedetemperatur zwischen 25°C und 120°C, vorzugsweise zwischen 40°C und 80°C, hat, zugibt. In dieser Lösung werden mindestens ein wasserlösliches, bei neutralem pH anionaktives Polymer, mindestens ein wasserlösliches, bei neutralem pH kationaktives Polymer, mindestens ein Feuchthaltemittel und ggfs. weitere Rezepturbestandteile gelöst, emulgiert oder suspendiert, wobei die Temperatur der Lösung während des Lösungsvorgangs die Siedetemperatur des flüchtigen Zusatzes nicht übersteigt. Die so erhaltene Mischung wird in eine Form gegossen oder auf einen Träger gestrichen und nach bekannten Verfahren auf eine definierte Restfeuchte getrocknet, wobei durch Entfernen des flüchtigen Zusatzes ein flexibler, hydrophiler Gelfilm entsteht, der in Wasser zwar quellbar, aber nicht löslich ist. Der die Reaktion von Polymeren unterschiedlicher Ladung verhindernde flüchtige Zusatz hat vorzugsweise basischen oder sauren Charakter.

Durch den flüchtigen Zusatz wird während des Lösungsvorgangs eine der beiden gegensätzlichen Polymerkomponenten durch reversible Salzbildung vorübergehend so inaktiviert, daß ihre Ladung nicht zum Tragen kommt. Durch das Entfernen des flüchtigen Zusatzes während der Trocknung wird die Salzbildung rückgängig gemacht und das Polymer bzw. die Polymere in seine/ihre Grundform überführt. Aus der Anziehung der gegensätzlichen Ladungen der Basispolymere resultiert dann die mechanische Stabilität des entstehenden Gelfilms. Flüchtige Zusätze mit basischem Charakter sind Ammoniumhydroxid oder primäre, sekundäre und tertiäre Amine mit Siedepunkten zwischen 25°C und 120°C wie Isopropylamin, Tertiär-butylamin, Diethylamin, Isobutylamin, Diisopropylamin, Isoamylamin oder Triethylamin.

Flüchtige Zusätze mit saurem Charakter sind Chlorwasserstoffsäure, Kohlensäure, Essigsäure oder Ameisensäure.

Für die Flexibilität des Gelfilms sind Feuchthaltemittel und Restwassergehalt verantwortlich. Der Gelfilm darf also nicht vollständig getrocknet werden, da das dazu führen würde, daß der Gelfilm spröde und brüchig wäre. Der optimale Restwasser- und Feuchthaltemittelgehalt muß für einen gegebenen Gelfilm durch Versuche ermittelt werden und ist bspw. auch abhängig von Art und Menge weiterer Hilfsstoffe und/oder Wirkstoffe, die der Gelfilm enthalten kann.

Als wasserlösliche oder wasserdispergierbare Hilfsstoffe kann der Gelfilm Weichmacher, Verdickungsmittel, Penetrationsbeschleuniger, Klebrigmacher, Konservierungsmittel, Desinfektionsmittel, pH-Regulatoren, Antioxidantien, Emulsionsstabilisatoren, Vernetzungsmittel, Füllstoffe und/oder Schaumstabilisierungsmittel enthalten.

Als Weichmacher kann der Gelfilm Citronensäureester wie Triethylcitrat oder Acetyltriethylcitrat, Weinsäureester wie Dibutyltartrat, Glycerinester wie Glycerindiacetat oder Glycerintriacetat, Phthalsäureester wie Dibutylphthalat oder Diethylphthalat und/oder hydrophile Tenside, vorzugsweise hydrophile, nichtionogene Tenside, wie bspw. Partialfettsäureester von Zuckern, Polyethylenglykolfettsäureester, Polyethylenglykolfettalkoholether oder Polyethylenglykol-sorbitan-fettsäureester enthalten.

Verdickungsmittel sind bspw. natürliche Substanzen und deren Derivate wie Kollagen, Galaktomannane, Stärken, Stärkederivate und -hydrolysate, Cellulosederivate wie Methylcellulose, Hydroxypropylcellulose (HPC), Hydroxyethylcellulose, Ethylhydroxyethylcellulose oder Hydroxypropylmethylcellulose, kolloidale Kieselsäuren, Quelltone, Zucker wie Lactose, Saccharose, Fructose oder Glucose, sowie synthetische Substanzen wie Polyvinylalkohol, Polyvinylpyrrolidon, Vinylpyrrolidon-Vinylacetat-Mischpolymerisate, Polyethylenglykole oder Polyproylenglykole. Bevorzugt eingesetztes Verdickungsmittel ist Kollagen.

Als Penetrationsbeschleuniger kann der Gelfilm bspw. Alkylsulfate, Alkylsulfonate, Alkaliseifen, fettsaure Salze von mehrwertigen Metallen, Betaine, Aminoxide, Fettsäureester, Mono-, Di- oder Triglyceride, langkettige Alkohole, Sulfoxide, Nicotinsäureester, Salicylsäure, N-Methylpyrrolidon, 2-Pyrrolidon oder Harnstoff enthalten.

Als Klebrigmacher kann der Gelfilm natürliche Harze oder Gummen wie Mastix, Damar, Elemi, Styrax, Euphorbium, Sandarak, Galbanum, Gummi Arabicum oder Karaya, modifizierte Naturharze wie Kolophoniumderivate, oder synthetische Harze oder Gummen, wie z.B. Polyacrylate, Polymethacrylate, Polyvinylether, Polyurethan, Polyisobutylene, Polyvinylester oder Silicone enthalten. In einer besonderen Ausführungsform umfaßt del Gelfilm als Klebrigmacher Zucker, Honig und/oder Pantothenylalkohol.

Als Konservierungsmittel kann der Gelfilm p-Cl-m-Kresol, Phenylethylalkohol, Phenoxyethylalkolhol, Chlorbutanol, 4-Hydroxybenzoesäuremethylester, 4-Hydroxybenzoesäurepropylester, Benzalkoniumchlorid, Cetylpyridiniumchlorid, Chlorhexidindiacetat oder -digluconat, Ethanol oder Propylenglykol enthalten.

Als Desinfektionsmittel kann der Gelfilm Halogene wie Polyvidon-Jod, Halogenverbindungen wie Natriumhypochlorit oder Tosylchloramid-Natrium, Oxidationsmittel wie Wasserstoffperoxid oder Kaliumpermanganat, Arylquecksilberverbindungen wie Phenylmercuriborat oder Merbromin, Alkylquecksilberverbindungen wie Thiomersal, Organozinnverbindungen wie Tri-n-butyl-zinnbenzoat, Silbereiweißverbindungen wie Silbereiweißacetyltannat, Alkohole wie Ethanol, n-Propanol oder Isopropanol, Phenole wie Thymol, o-Phenylphenol, 2-Benzyl-4-Chlorphenol, Hexachlorophen oder Hexylresorcin oder organische Stickstoffverbindungen wie 8-Hydroxychinolin, Chlorquinaldol, Clioquinol, Ethacridin, Hexetidin, Chlorhexidin oder Ambazon enthalten.

Als pH-Regulatoren kann der Gelfilm bspw. Glycinpuffer, Citratpuffer, Boratpuffer, Phosphatpuffer oder Citronensäure-Phosphat-Puffer enthalten.

Als Antioxidantien kann der Gelfilm bspw. Ascorbinsäure, Ascorbylpalmitat, Tocopherolacetat, Propylgallat, Butylhydroxyanisol oder Butylhydroxytoluol enthalten.

Als Emulsionsstabilisatoren kann der Gelfilm nichtionogene Emulgatoren, wie z.B. höhere Fettalkohole, Partialfettsäureester mehrwertiger Alkohole, Partialfettsäureester von Zuckern, Polyethylenglykolfettsäureester, Polyethylenglykolfettalkoholether, Polyethylenglykol-sorbitan-fettsäureester, aber auch amphotere Emulgatoren, wie z.B. Phospholipide, Ampholytseifen oder Eiweiße, kationaktive Emulgatoren, wie z.B. quartäre Ammoniumverbindungen oder Pyridiniumverbindungen, und anionaktive Emulgatoren, wie z.B. fettsaure Salze von mehrwertigen Metallen, Alkylsulfate und Alkylsulfonate enthalten.

Als Füllstoffe kann der Gelfilm z.B. mikrokristalline Cellulose, Aluminiumoxid, Zinkoxid, Titandioxid, Talkum, Siliciumdioxid, Magnesiumsilikat, Magnesium-Aluminiumsilikat, Kaolin, hydrophobe Stärke, Calciumstearat oder Calciumphosphat enthalten.

Als Vernetzungsmittel kann der Gelfilm z.B. mehrwertige Kationen, wie Zink-, Magnesium-, Aluminium-, Calcium-, Chrom, Kupfer- oder Eisenionen, oder Anionen mehrwertiger Säuren, wie Sulfat-, Phosphat-, Silikat-, Borat-, Tartrat- oder Citrationen enthalten.

Der Gelfilm kann eine lipophile, innere Phase aufweisen, wobei diese lipophile Phase unzerteilt und kohärent oder zerteilt, vorzugsweise in kleine Partikel oder Tröpfchen, vorliegen kann. Die lipophile innere Phase kann bspw. aus natürlichen, halbsynthetischen oder synthetischen Fetten und Ölen wie Olivenöl, Ricinusöl, Erdnußöl, Sojaöl, Leinöl, Sesamöl, Jojobaöl, Avocadoöl, hydriertes Erdnußöl, hydriertes Ricinusöl, Triglyceridgemische (Miglyol^{R}, Softisan^{R}) oder Silikonöle, natürlichen, halbsynthetischen oder synthetischen Wachsen wie Bienenwachs, Wollwachs, Erdwachs, Walrat, Ölsäureoleylester, Isopropylpalmitat, Isopropylmyristat, Ethyloleat, Cetylpalmitat oder Cetylstearat, Fettalkohole wie Dodecylalkohol oder Cetylalkohol, Fettsäuren wie Myristinsäure, Ölsäure oder Linolsäure, propoxylierte, ethoxylierte oder sulfatierte Fettalkohole, Fettsäurealkylamide, Fettsäure-Eiweiß-Kondensationsprodukte, Phospholipide, Sterine oder Kohlenwasserstoffe wie Paraffine oder Paraffinöle bestehen. Liegt die lipophile innere Phase des Gelfilms in zerteilter Form vor, so werden zur Stabilisierung der zerteilten Phase Hilfsstoffe wie bspw. Emulgatoren und/oder Schutzkolloide zugesetzt.

In einer besonderen Ausführungsform wird die Zusammensetzung des flexiblen, hydrophilen Gelfilms so gewählt, daß der Film transparent ist.

Während des Lösevorgangs der Rezepturbestandteile kann in die Masse Luft mit eingerührt werden, so daß der nach dem Ausstreichen oder Ausgießen erhaltene Gelfilm eine poröse Struktur hat. Herstellung und Stabilisierung der porösen Struktur können z.B. durch die Zugabe von Schäumungsmitteln wie Saponinen, Alginsäureestern, Aminoxiden oder Fettaminoxiden unterstützt werden.

Die Zusammensetzung des flexiblen, hydrophilen Gelfilms kann so gewählt werden, daß der Gelfilm biologisch abbaubar ist, wobei unter biologischem Abbau bspw. die Einwirkung von UV-Licht, die Einwirkung von Mikroben oder die Einwirkung von Enzymen verstanden wird.

Der flexible, hydrophile Gelfilm kann mehrschichtig aufgebaut sein. Es können bspw. zumindest zwei Gelfilme nach bekannten Verfahren zu einem Verbund zusammengefügt werden. Ebenso ist es möglich, einen oder mehrere Gelfilme mit einem oder mehreren lipophilen Polymerfilmen zu einem Verbund beliebigen Aufbaus zusammenzufügen. Die einzelnen Schichten des Verbunds können dabei in sich zusammenhängend und kohärent sein, sie können aber auch in Segmente zerteilt sein, die von der darüber oder darunter liegenden Schicht umgeben oder umschlossen werden. So kann bspw. eine lipophile Kleberschicht zusammenhängend oder punkt- bzw. rautenförmig zerteilt auf einen Gelfilm aufgebracht sein, wobei der Gelfilm die Zwischenräume zwischen den lipophilen Segmenten ausfüllen kann. In einer anderen Ausführungsform wird der Gelfilm mit textilen Geweben, Fasergeflechten und/oder natürlichen oder synthetischen Schäumen kombiniert. Bei diesem Mehrschichtverbund können die einzelnen Schichten nach bekannten Verfahren getrennt hergestellt und anschließend zusammengebracht werden. Die Schichten können sich aber auch gegenseitig durchdringen, so bspw. wenn die Gelmasse vor der Filmbildung auf ein Gewebe, ein Fasergeflecht oder einen Schaum gegeben wird und die Filmbildung erst nach Einziehen der Gelmasse in das Gewebe oder den Schaum erfolgt.

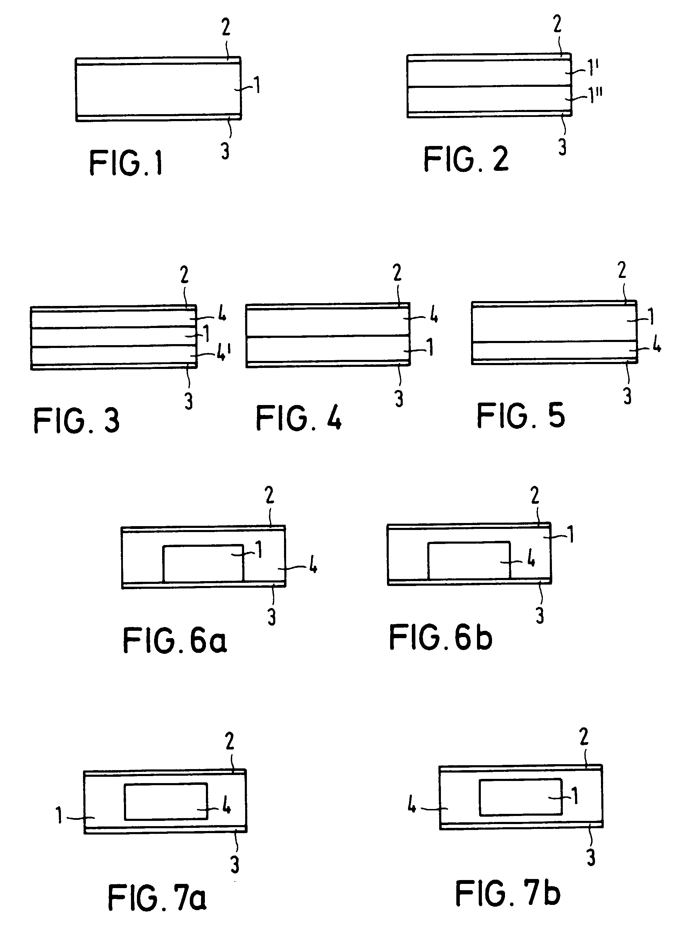

Der flexible, hydrophile Gelfilm kann verwendet werden zur Herstellung von Vorrichtungen zur Erzeugung von Wechselwirkungen zwischen dem Gelfilm und einem festen, flüssigen oder gasförmigen Substrat. Die Wechselwirkung zwischen Gelfilm und Substrat kann durch direkten Kontakt oder indirekten Kontakt zwischen Gelfilm und Substrat erzeugt werden; bei indirektem Kontakt wird die Wechselwirkung vermittelt durch Teile der Vorrichtung wie bspw. Klebschichten, Steuerelemente oder durchlässige Trennelemente. Zum Schutz vor Austrocknung und dem Einwachsen von Keimen wird der Gelfilm nach bekannten Verfahren mit einer undurchlässigen Rückschicht und auf der gegenüberliegenden Seite mit einer ablösbaren undurchlässigen Schutzschicht versehen (Fig. 1).

Rückschicht und Schutzschicht sind vorzugsweise Folien aus bspw. Polyamid, Polycarbonat, Polyester, Polyethylen, Polypropylen, Polyvinylchlorid, Polyvinylester, Polyurethan, Polystyrol, Polytetrafluorethyllen, Cellophan, Celluloseacetat, Aluminium oder sind Verbundfolien aus den vorgenannten Materialien. Der Gelfilm wird dann bspw. in für die Verwendung geeignete Abschnitte zerteilt und konfektioniert. Die Zerteilung des Gelfilms kann aber auch durch den Anwender selbst vorgenommen werden. Der Gelfilm befindet sich bspw. aufgerollt oder zusammengefaltet in einem Behältnis, und der Anwender kann sich selbst Segmente, bspw. durch Schnitt, abtrennen, die eine dem Verwendungszweck entsprechende Fläche haben. Die Fläche der Segmente kann aber auch mittels Sollbruchlinien, hergestellt bspw. durch Anstanzen oder Anschneiden des Gelfilms, bereits vorgegeben werden, so daß der Anwender die einzelnen Segmente abreißen kann, ohne technische Hilfsmittel dazu verwenden zu müssen. Die Zerteilung des Gelfilms kann aber auch dadurch vorgenommen werden, daß die Gelmasse vor der Filmherstellung in Formen ausgegossen wird, durch die die Fläche des fertigen Films vorgegeben wird. In diesem Fall kann auf das Aufbringen einer undurchlässigen Schutzschicht und/oder undurchlässigen Rückschicht auf den Gelfilm verzichtet werden, sofern die Gußform Teil der Konfektionierung ist und nach der Filmherstellung bspw. mit einer Siegelfolie dicht verschlossen wird.

Wird der Gelfilm verwendet zur Herstellung von Vorrichtungen zur Erzeugung von Wechselwirkungen zwischen dem Gelfilm und einem festen Substrat oder einem flüssigen Substrat begrenzter Fläche das von festem Substrat umgeben ist, weist diese Vorrichtung zur Herstellung des Kontaktes zwischen Gelfilm und Substrat eine Befestigungseinrichtung auf. Diese Befestigungseinrichtung kann bspw. eine Binde oder Bandage, ein herkömmliches Heftpflaster oder eine Klebefolie sein.

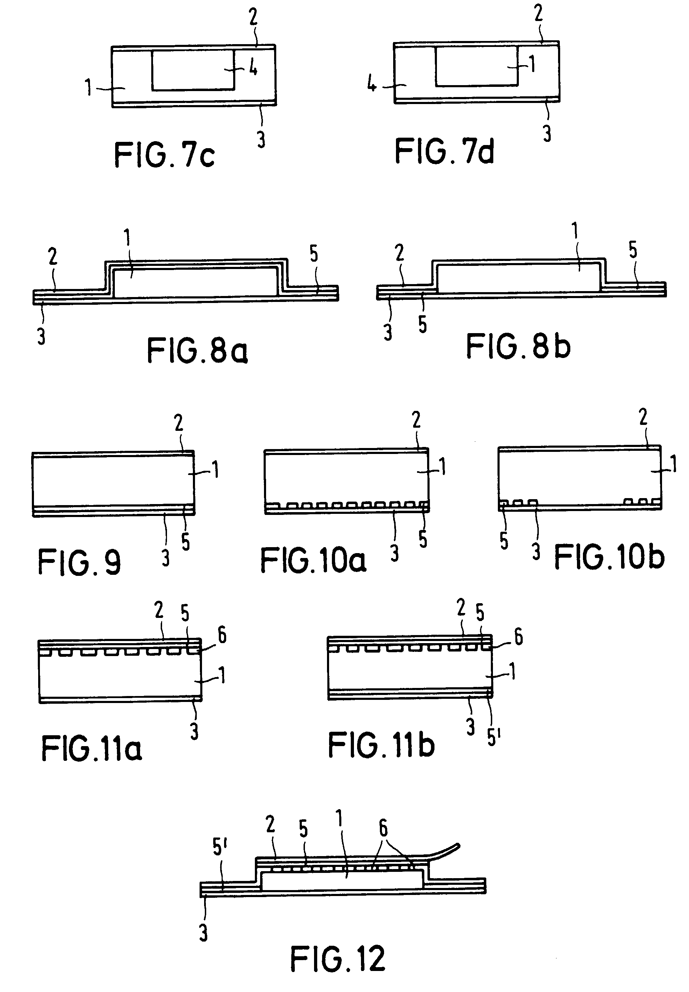

Die Befestigung auf dem Substrat kann auch durch den Gelfilm selbst erfolgen, sofern er Klebrigmacher enthält oder, wenn er mehrschichtig aufgebaut ist, zumindest Teile der Kontaktschicht zum Substrat klebende Eigenschaften haben (Fig. 9, 10). Eine weitere Möglichkeit zur Befestigung der Vorrichtung besteht darin, daß die Rückschicht größere Flächenabmessungen als das Gelfilmsegment hat und daß zumindest die überstehenden Teile der Rückschicht selbstklebend ausgerüstet sind und daß damit die Vorrichtung auf dem Substrat befestigt wird (Fig. 8).

Feste Substrate, mit denen der Gelfilm in Wechselwirkung treten kann, und die möglichen Wechselwirkungen können sehr vielfältiger Natur sein. So kann bspw. die Wechselwirkung zwischen Gelfilm und festen Substraten darin bestehen, daß der Gelfilm als Haftkleber zur Verankerung von Vorrichtungen auf festen Oberflächen verwendet wird.

In einer bevorzugten Ausführung ist das Substrat, mit dem der Gelfilm in Wechselwirkung tritt, geschädigte Haut.

Die Zusammensetzung des Gelfilms, der in Wechselwirkung mit durch Verbrennungen, Verbrühungen, Verätzungen, Operationswunden, Druckgeschwüren, Beingeschwüren oder auch Bagatellverletzungen wie Schürf- und Kratzwunden geschädigter Haut steht, wird so gewählt, daß alle Komponenten optimale Gewebeverträglichkeit aufweisen, damit die natürlich ablaufenden Wundheilungsprozesse gefördert und nicht etwa gestört oder unterbrochen werden. Als kationaktive Polymerkomponente wird vorzugsweise Typ-A-Gelatine verwandt, da Gelatine in der Wundbehandlung als Absorptionspolymer mit blutstillender Wirkung seit langem bekannt ist. Als anionaktive Komponenten werden für diese Verwendung vorzugsweise Natriumcarboxymethylcellulose oder niedrig veresterte Pektine verwandt. Die Vorrichtung, die die Wechselwirkung zwischen Gelfilm und geschädigtem Gewebe erzeugt bzw. ermöglicht, ist vorzugsweise transparent, da dadurch die Beobachtung des geschädigten Gewebes ermöglicht wird, ohne daß dafür die Vorrichtung entfernt werden muß.

Der Einstellung des Absorptionsvermögens für Wundabsonderungen und der Absorptionskinetik des Gelfilms kommt besondere Bedeutung zu. Die Basispolymere Typ-A-Gelatine und Natriumcarboxymethylcellulose z.B. weisen in Kombination ein rapides und hohes Absorptions- und Quellvermögen auf. Daher müssen Verlauf und Ausmaß der Absorption durch Einarbeitung weiterer Additive beeinflußt werden, wobei der Gehalt an zusätzlichen Additiven so zu wählen ist, daß dadurch der Zusammenhalt und damit die Handhabung des Gelfilms im gequollenen Zustand nicht leiden.

Bevorzugt eingesetzt werden physiologisch unbedenkliche Polymere, die in der Wundbehandlung in anderer Form schon lange eingesetzt werden, wie Polyvinylpyrrolidon, Polyvinylalkohol und insbesondere Kollagen.

Durch den Einsatz der genannten Polymere gelingt es, das Absorptionsverhalten des Gelfilms so zu steuern, daß hohe Flüssigkeitsmengen über einen vorgegebenen Zeitraum mit einer vorgegebenen Geschwindigkeit aufgesaugt und gespeichert werden können, ohne daß die Wunde ausgetrocknet wird. Insbesondere die Einarbeitung von Kollagen in den Gelfilm ist von Vorteil, da das Kollagen aus dem Gelfilm unter der Anwendung herausgelöst wird und in der Wunde als aktives Prinzip zur Förderung der Wundheilung zur Verfügung steht.

Eine weitere Möglichkeit zur Steuerung des Absorptionsverhaltens und -vermögens besteht darin, dem Gelfilm durch gezielte Schäumung der Masse vor dem Ausstreichen eine poröse Struktur zu verleihen. Durch die nach dem Trocknen im Gelfilm vorhandenen Hohlräume wird das Speichervermögen für Wundexsudat erhöht und durch die feinen Kapillaren, die den Gelfilm durchziehen, die Aufsauggeschwindigkeit gesteigert.

Ebenso ist es möglich, den Gelfilm mehrschichtig aufzubauen und durch die Kombination mehrerer homogener Schichten unterschiedlicher Zusammensetzung oder Kombination homogener und poröser Schichten die gewünschten Eigenschaften einzustellen.

Wird der Gelfilm mit einer Rückschicht versehen, so kann diese Rückschicht in einer besonderen Ausführungsform wasserdampfdurchlässig sein, damit die von der Wunde aufgenommene Flüssigkeit, die durch Diffusionstransport im Gelfilm an die rückseitige Grenzfläche der Wundauflage gelangt, von dort über die Rückschicht an die Umgebung abgegeben wird, so daß auf der Wunde kein Flüssigkeitsstau entsteht, der den Ablauf der Wundheilungsprozesse hindert.

Falls die Transparenz der Vorrichtung, die den Gelfilm in Kontakt mit dem geschädigten Gewebe bringt, von untergeordneter Bedeutung ist, kann der Gelfilm auch mit Schäumen wie bspw. Polyurethanschäumen, oder mit textilen Flächengebilden zu einem Mehrschichtverbund zusammengefügt sein. Wird die Haftung der Vorrichtung auf dem Substrat bei dieser Verwendung durch den Gelfilm selbst bewirkt, so werden als Klebrigmacher vorzugsweise Zucker, Honig, Pantothenylalkohol oder natürliche Harze und Gummen wie bspw. Mastix, Gummi Arabicum oder Karaya eingesetzt.

In einer anderen bevorzugten Ausführung ist das Substrat, mit dem der Gelfilm in Wechselwirkung tritt, intakte Haut. Der Gelfilm kann bspw. Teil von Vorrichtungen sein, die in der Kosmetik als Masken oder Filme zur Behandlung bspw. von gealterter Haut, Falten, unreiner Haut, zur Körperpflege, zur Haarentfernung, zur Verminderung der Schweißabsonderung oder zum Lichtschutz eingesetzt werden.

Eine bevorzugte Wechselwirkung zwischen Gelfilm und intakter Haut ist die dermale, intradermale oder transdermale Abgabe von Wirkstoffen.

Zur dermalen Behandlung lokaler Hauterkrankungen werden Lokalanästhetika, Lokalantibiotika, Antimykotika, Antihistaminika und juckreizstillende Arzneistoffe, Keratolytika und ätzende Arzneistoffe, Antiskabiesmittel, Steroide, sowie verschiedene Substanzen zur Behandlung von Akne, Psoriasis oder Lichtdermatosen verwendet.

Zu den Wirkstoffen, die intradermal appliziert werden, gehören z.B. steroidale und nichtsteroidale Antirheumatika, Antiphlogistika, durchblutungsfördernde Substanzen oder Vasoprotektoren und -konstriktoren zur Behandlung von Gefäßerkrankungen. Zu den transdermal applizierbaren Wirkstoffen gehören z.B. Neuroleptika, Antidepressiva, Tranquillantien, Hypnotika, Psychostimulantien, Analgetika, Muskelrelaxantien, Antiparkinsonmittel, Ganglienblocker, Sympathomimetika, Alpha-Sympatholytika, Beta-Sympatholytika, Antisympathotonika, Antidiabetika, Koronartherapeutika, Antihypertonika, Antiasthmatika oder Diuretika.

Es ist bekannt, daß die Durchlässigkeit der Haut für Wirkstoffe abhängig ist unter anderem von der Dicke, dem Lipidgehalt und dem Wassergehalt der Hornschicht. Ebenso ist bekannt, daß die Erhöhung des Wassergehalts der Hornschicht eine Quellung bewirkt, die die Permeabilität der Haut für viele Wirkstoffe um den Faktor 4-5 erhöht. Diese Quellung der Hornschicht kann bei Applikation des erfindungsgemäßen Gelfilms durch die Abgabe von Wasser aus dem Gelfilm an die Haut erreicht werden.

Die Funktionen, die der Gelfilm bei dieser Verwendung erfüllen kann, sind vielfältig. Im einfachsten Fall besteht eine Vorrichtung zur Abgabe von Wirkstoffen an die Haut aus einer für Wirkstoff undurchlässigen Rückschicht, einem Substanzreservoir und einer Schutzschicht, die vor Applikation entfernt wird (Fig. 1).

Wird der Gelfilm als einschichtiges Substanzreservoir verwendet, so ist die Wirkstofffreigabe neben der Hydratisierung der Haut durch zugeführtes Wasser abhängig von der Filmdicke, dem Wirkstoffgehalt im Gelfilm und der thermodynamischen Aktivität von Wirkstoff im Gelfilm, wobei es verschiedene Möglichkeiten gibt, in Abhängigkeit der Substanzeigenschaften die thermodynamische Aktivität im Gelfilm zu verändern.

Ist die Löslichkeit von Wirkstoff z.B. pH-abhängig, so kann durch die Einarbeitung von pH-Regulatoren wie z.B. Puffersystemen die Löslichkeit der Substanz erhöht oder erniedrigt und damit die Freisetzung reguliert werden. Sehr viele Wirkstoffe sind basisch und zeigen bei neutralem pH aufgrund reduzierter Ionisation die geringste Löslichkeit. Hier kann durch Zugabe saurer Additive die Löslichkeit erhöht werden. Ebenso ist es möglich, bei sehr gut wasserlöslichen Wirkstoffen, deren Freisetzung aus hydrophilen Matrices prinzipiell verzögert ist, durch Auswahl der Gelfilmzusammensetzung, Einstellung des pH-Wertes und Reduzierung des Wassergehaltes die Löslichkeit zu verringern.

Eine andere Möglichkeit zur Beeinflußung der thermodynamischen Aktivität und damit der Regulierung der Freisetzung, z.B. bei extrem lipophilen Wirkstoffen, besteht darin, den Gelfilm mit einer lipophilen inneren Phase zu versehen.

Dieser Film ist dann praktisch eine mechanisch stabile, filmförmige Creme.

Die Zusammensetzung der inneren Phase, insbesondere die Zusammensetzung einer unter Umständen notwendigen emulsionsstabilisierenden Komponente, wird so gewählt, daß eine hohe Affinität zu den Hautlipiden gegeben ist. Dadurch wird erreicht, daß bei Kontakt mit der Haut sich die Lipidvesikel durch Destabilisierung der Emulsion öffnen und eingearbeitete Substanz an die Haut abgegeben wird.

Ein weiterer vorteilhafter Effekt des Gelfilms ist, daß er durch Applikation aktiviert werden kann, d.h., daß bei entsprechender Zusammensetzung durch Erwärmung des Gelfilms auf Körpertemperatur die thermodynamische Aktivität von Wirkstoff oder lipophiler innerer Phase erhöht werden kann.

Die Abgabeeinheit einer Vorrichtung zur dermalen, intradermalen oder transdermalen Applikation ist aber nicht unbedingt ein einschichtiges Reservoir, sondern sie kann auch mehrteilig sein. So können z.B. mehrere Gelfilme, die Wirkstoffe enthalten, zu einem Laminat verbunden sein (Fig. 2). Die Möglichkeiten zur Steuerung bestehen z.B. in unterschiedlicher Zusammensetzung der einzelnen Schichten oder unterschiedlicher Wirkstoffbeladung.

In einem Mehrschichtverbund kann ein wirkstofffreier Gelfilm z.B. als Sperrschicht zwischen lipophilen Schichten fungieren, die erst durch Erwärmung auf Körpertemperatur permeabel wird (Fig. 3), oder zwischen lipophilem Reservoir und Haut als Steuermembran für die Abgabe von Wirkstoff dienen (Fig. 4). Ebenso ist es möglich, daß der Gelfilm oder ein Gelfilmverbund Träger von Wirkstoff ist und eine wirkstofffreie lipophile Schicht als Steuermembran zwischen Gelreservoir und Haut dient (Fig. 5), wobei die lipophile Schicht, wenn sie selbstklebend ausgerüstet ist, auch zur Verankerung des Gelfilms auf der Haut dient. In einer anderen Ausführungsform ist der Gelfilm in Plättchenform zumindest teilweise von lipophilem Polymerfilm umgeben oder umgibt selbst zumindest teilweise ein lipophiles Element. Hydrophiler und lipophiler Teil der Vorrichtung können dabei Hautkontakt haben (Fig. 6). Falls beide Teile dieselbe Wirksubstanz oder dieselben Wirksubstanzen enthalten, kann, bedingt durch die sehr unterschiedliche chemische Natur, ein Teil der Vorrichtung sehr schnell Wirksubstanz an die Haut abgeben als Initialdosis zur raschen Erzielung therapeutisch wirksamer Blutspiegel.

Der andere Teil der Vorrichtung liefert verzögert über einen längeren Zeitraum die Erhaltungsdosis zur Aufrechterhaltung der Blutspiegel.

Es kann in den unterschiedlichen Teilen der Vorrichtung aber auch unterschiedlicher Wirkstoff enthalten sein. Bei Erkrankungen, die üblicherweise mit einer Kombination von Wirkstoffen therapiert werden, ist die dermale, intradermale oder transdermale Applikation dieser Kombinationen oftmals nicht möglich, da z.B. Pharmakokinetik und Pharmakodynamik der Wirkstoffe bei Applikation auf der Haut stark voneinander abweichen, die Wirkstoffe aufgrund chemischer Unterschiede nicht in einem Reservoir unterzubringen sind oder zumindest ein Wirkstoff nicht die gewünschte Freisetzung zeigt. Die Kombination eines hydrophilen Gelfilms mit einem Emulsionsgelfilm oder einem herkömmlichen lipophilen Polymerfilm, die jeder für sich einen anderen Wirkstoff enthalten, macht es möglich, daß für jeden Wirkstoff eine spezifische Formulierung gewählt werden kann, die die gewünschte Freisetzung liefert. Der Vorteil dieser Ausführungsform liegt darin, daß für die Kombinationstherapie mit mehreren Arzneistoffen nicht auch gleichzeitig mehrere Vorrichtungen appliziert werden müssen. Außerdem ist es möglich, über die Kombination des Gelfilms mit lipophileren Filmen z.B. unterschiedliche Wirkstoff zu unterschiedlichen Zeitpunkten freizusetzen. Aus einem Teil der Vorrichtung kann ein Wirkstoff sehr schnell freigesetzt werden und erreicht schnelle therapeutisch wirksame Blutspiegel, die nach einer bestimmten Zeit, vorzugsweise nach 4-12 Stunden, wieder abnehmen.

Der andere Teil der Vorrichtung stellt eine Retardformulierung dar, die einen anderen Wirkstoff mit verzögerter Freisetzung langsam an die Haut abgibt, so daß die therapeutisch notwendige Konzentration erst nach einiger Zeit, vorzugsweise nach 4-12 Stunden, erreicht wird. Durch die alternierende Freisetzung von Wirkstoffen kann z.B. auch gezielter auf Therapieschemata eingegangen werden, wie sie bei peroraler Verabreichung von Arzneistoffen üblich sind unter Vermeidung der Nachteile peroraler Arzneiformen. So kann bspw. auch durch die alternierende Freisetzung von Nitraten und Calciumantagonisten oder Nitraten und Beta-Rezeptorenblockern eine wirksame Angina-Pectoris-Prophylaxe betrieben werden, ohne daß eine Toleranzentwicklung gegen Nitrate auftritt. Bei all diesen Formen muß durch Auswahl der Formulierungen oder durch zusätzliche Trennschichten sichergestellt sein, daß es durch Wechselwirkungen zwischen hydrophilen und lipophilen Vorrichtungsteilen nicht zu Veränderungen des Freisetzungsverhaltens während der Lagerung kommt.

In einer anderen Ausführungsform umschließt der Gelfilm einen lipophilen Wirkstoffspeicher oder der Gelfilm ist Wirkstoffspeicher in einem lipophilen Film (Fig. 7). In beiden Fällen hat der Wirkstoffspeicher keinen direkten Kontakt zur Haut; seine Funktion liegt darin, über einen langen Zeitraum kontrolliert Wirkstoff an den umgebenden Film abzugeben. Während der Lagerung stellt sich ein Wirkstoffgleichgewicht zwischen Speicherelement und umgebendem Element ein, das von der Sättigungslöslichkeit des Wirkstoffes im umgebenden Film abhängig ist. Nach der Applikation penetriert Wirkstoff aus dem umgebenden Element in die Haut und in Abhängigkeit von der Diffusionsgeschwindigkeit des Wirkstoffes aus dem Speicher in das umgebende Element, der Diffusionsgeschwindigkeit des Wirkstoffes im umgebenden Element und der Penetrationsrate in die Haut wird aus dem Speicher über einen langen Zeitraum kontinuierlich Wirkstoff nachgeliefert, bis die Sättigungslöslichkeit des Wirkstoffes im Speicher unterschritten wird.

In einer weiteren bevorzugten Ausführung ist das Substrat, mit dem der Gelfilm in Wechselwirkung tritt, Schleimhaut.

Der Gelfilm dient dabei als Träger für lokal oder systemisch wirksame Substanzen zur Abgabe an Schleimhäute, wie Augen-, Mund- und Nasenschleimhaut, rektale, vaginale oder uterine Schleimhaut. Die Haftung an Schleimhäuten, allgemeinen als Bioadhäsion bezeichnet, wird bewirkt durch Grenzflächenkräfte zwischen der Schleimhaut und dem Gelfilm, durch die der Film für einen vorbestimmten Zeitraum mit dem Gewebe verbunden wird.

Der Gelmasse werden vor der Filmherstellung Polymere zugesetzt, die bekanntermaßen eine hohe Affinität zu Schleimhäuten aufweisen und über einen langen Zeitraum eine ausgezeichnete Haftung zeigen, wie z.B. Carboxymethylcellulose, Polyacrylsäure (Carbopol^{R}, Goodrich), Traganth, Natriumalginat, Hydroxyethylcellulose, Hydroxypropylcellulose oder Karaya. Nach Applikation nimmt der Gelfilm Körpersekrete auf, quillt und setzt Wirkstoff über längere Zeit frei. Die Freisetzung erfolgt entweder durch Erosion des Gelfilms oder durch gesteuerten Abbau des Gelgerüstes oder eine Mischung beider Mechanismen. Spezielle Verwendungszwecke sind z.B. die okulare Applikation von Pilocarpin oder Lokalantibiotika sowie z.B. bei vaginaler oder intrauteriner Applikation die Langzeitabgabe von Östrogenen und/oder Gestagenen zur Empfängnisverhütung.

Bei der bukkalen oder sublingualen Applikation des Gelfilms kann zum einem Wirkstoff über die Mundschleimhaut an den Systemkreislauf abgegeben werden. In diesem Fall besitzt der Gelfilm eine Rückschicht, die verhindert, daß größere Mengen Wirkstoff durch Speichel aus dem Gelfilm herausgelöst und nach Verschlucken gastrointestinal resorbiert werden.

Zum anderen können mit der bukkalen Applikation des Gelfilms bspw. aber auch Fluoride zur Kariesprophylaxe oder Wirkstoffe zur Behandlung von Erkrankungen, bspw. Entzündungen im Mund- und Rachenraum freigesetzt werden. Eingesetzt werden bspw. Lokalantiseptika wie Chlorhexidin, Benzalkonium, Hexetidin, Dequaliumchlorid, Acriflavin oder Ethacridin, Lokalanästhetika wie Benzocain oder Lidocain, Lokalantibiotika wie Gramicidin oder Tyrothricin, Adstringentien wie z.B. Aluminiumsalze oder Pflanzenextrakte aus Salbei, Myrrhe oder Benzoe.

Das Substrat, mit dem der Gelfilm in Wechselwirkung tritt, kann aber auch eine Pflanzenoberfläche sein. So können bspw. oberflächliche, lokal begrenzte Pflanzenkrankheiten mit Wirkstoffen behandelt werden, die aus dem Gelfilm an die Pflanzenoberfläche abgegeben werden. Ebenso können Wirkstoffe mit systemischer Wirksamkeit, die dem Schutz der Pflanze vor Krankheitserregern und Schädlingen oder der Behandlung von Pflanzen nach erfolgter Infektion und bereits vorhandenem Befall, der Wachstumsregulierung oder der Ernährung der Pflanze dienen, aus dem Gelfilm an die Pflanzenoberfläche, wie bspw. die der Blätter, des Stamms oder der Wurzeln, abgegeben werden. Der Wirkstoff penetriert nach der Abgabe aus dem Gelfilm in epidermale und subepidermale Gewebe der Pflanze, tritt in die Stofftransportsysteme der Pflanze über und wird nach der Resorption systemisch in der Pflanze verteilt.

Die systemischen Wirkstoffe können bspw. Fungizide wie Bitertanol, Fenarimol, Triforin, Aluminiumfosetyl oder Tridemorph, Insektizide wie Nicotin, Demeton, Dimethoat, Fenthion oder Menazon, oder Pflanzenhormone wie Auxine, Gibberelline, Cytokinine oder Abscisinsäure sein. Der Gelfilm kann aber auch der Konservierung isolierter Pflanzenteile, bspw. durch Umwickeln der Pflanzenteile, oder der Versorgung von Schnittblumen mit Nährstoffen und Wasser durch Umwickeln der Schnittstellen dienen.

Auch die Wechselwirkungen zwischen Gelfilm und flüssigem Substrat können sehr vielfältiger Natur sein. So kann der Gelfilm bspw. Teil einer Vorrichtung sein, die als Badezusatz dient. Nach Entfernen der Schutzschicht und/oder der Rückschicht und dem Einbringen der Vorrichtung in das Bad können aus dem Gelfilm an das flüssige Substrat bspw. Duftstoffe, Farbstoffe, Reinigungsmittel, Körperpflegemittel, Mittel zur Behandlung von Hautfunktionsstörungen, juckreizstillende Wirkstoffe wie Kamille, Schwefel, Weizenkleie oder Milcheiweiß, Stoffe mit anregender Wirkung wie Lavendel, Rosmarin oder sauerstofffreisetzende Verbindungen, Stoffe mit beruhigender Wirkung wie Baldrian-, Hopfen- oder Melisseauszüge bzw. -inhaltsstoffe, Wirkstoffe zur Linderung von rheumatischen Beschwerden wie Salicylsäure und Salicylsäureester, Nicotinsäureester, Auszüge und Inhaltsstoffe von Arnika, Calendula oder Capsicum, oder Wirkstoffe zur Linderung von Erkältungsbeschwerden wie Auszüge und Inhaltsstoffe von Eucalyptus, Fichtennadel, Latschenkiefer oder Thymian.

Der Gelfilm kann aber auch Teil von Vorrichtungen sein, die in Aquarien eingebracht werden und bei denen die Wechselwirkung zwischen Gelfilm und flüssigem Substrat bspw. in der Freisetzung von Wirkstoffen zur Verhütung oder Behandlung von Fischkrankheiten oder zur Bekämpfung von Algenwachstum besteht.

Vorrichtungen zur Erzeugung von Wechselwirkungen zwischen Gelfilm und gasförmigen Substraten haben in der Regel undurchlässige Rückschichten und Schutzschichten. Der Gelfilm enthält vorzugsweise Wirkstoffe, die bei Raumtemperatur flüchtig sind und die nach Entfernen der Schutzschicht und/oder der Rückschicht an das gasförmige Substrat abgegeben werden. Zur Steuerung der Wirkstoffabgabe können sich zwischen Rückschicht und Gelfilm und/oder zwischen Schutzschicht und Gelfilm poröse Membranen befinden.

Eine mögliche Verwendung einer solchen Vorrichtung ist bspw. die Behandlung von Erkältungskrankheiten mit einem Gelfilm, der ätherische Öle wie bspw. Anisöl, Fenchelöl, Eucalyptusöl, Thymianöl, Kamillenöl, Pfefferminzöl, Campher, Terpentinöl, Latschenkieferöl oder aber einzelne flüchtige Inhaltsstoffe ätherischer Öle wie bspw. Menthol, Eucalyptol oder Phellandren, umfaßt. Der Gelfilm kann nach Entfernen der Rückschicht und Freilegen der Abgabefläche bspw. vor dem Einschlafen auf das Kopfkissen gelegt werden, und während der Nacht wird flüchtiger Wirkstoff an die Umgebung abgegeben und eingeatmet. Der Gelfilm kann aber auch eine haftklebene Fixiereinrichtung umfassen (Fig. 11,12) und wird nach Entfernen von Rückschicht und Schutzschicht im oberen Brustbereich auf die Haut appliziert. Durch das Erwärmen auf Körpertemperatur wird die Abgabe flüchtigen Wirkstoffs noch verstärkt und damit die eingeatmete Menge erhöht. Der Vorteil gegenüber den handelsüblichen Gelen, Balsamen und Einreibungen liegt darin, daß durch die Vorrichtung eine gesteuerte Abgabe von Substanz über einen längeren Zeitraum ermöglicht wird und daß ein Verkleben mit Kleidung oder Bettwäsche vermieden wird. In einer anderen Ausführungsform umfaßt der Gelfilm beruhigend wirkende oder schlaffördernde flüchtige Wirkstoffe wie bspw. Inhaltsstoffe oder Extrakte der Melisse, des Hopfens oder des Baldrians. Die Anwendung erfolgt wie zuvor beschrieben. Die Wechselwirkung zwischen Gelfilm und gasförmigen Substrat kann aber auch darin bestehen, daß aus dem Gelfilm flüchtige Wirkstoffe zum Schutz gegen Stechmücken, Stubenfliegen, Bremsen oder Wespen an die Luft abgegeben werden. Diese Wirkstoffe können Insektizide wie bspw. Pyrethrine, synthetische Pyrethroide oder Propoxur sein oder Repellentien wie bspw. Diethyltoluamid, Dimethylphthalat, Lorbeeröl, Eucalyptusöl oder Anisöl.

### Beispiele 1-18

Zu dem Lösemittel Wasser (Beispiele 1-8, 13-18) bzw. der Lösemittelmischung Wasser/Ethanol (Beispiele 9-12) wird in einem dicht verschließbaren Gefäß der Lösungsvermittler (Ammoniumhyroxid in Beispielen 1-12, Eisessig in Beispielen 13-18) gegeben, und die Mischung wird auf 65°C erwärmt. Anionaktives Polymer, kationaktives Polymer und die übrigen Rezepturbestandteile werden nacheinander zugegeben, und es wird jeweils im geschlossenen Gefäß solange gerührt und homogenisiert bis die jeweilige Substanz klar gelöst ist. Die Masse wird bis auf eine Temperatur von ca. 45°C kaltgerührt (Beisp. 1-11, 13-18) und bei dieser Temperatur auf einen Träger, der Rückschicht, Schutzschicht oder Zwischenabdeckung sein kann, in gewünschter Schichtdicke gestrichen oder beschichtet und im Trockenkanal bei 80°C auf eine Restfeuchte von ca. 25% getrocknet.

Beispiel 12 unterscheidet sich im Herstellverfahren dadurch, daß in allen Schritten bis zur Trocknung eine Temperatur von 30°C nicht überschritten wird und die Masse nach dem Ausstreichen bei 60°C getrocknet wird.

Die so erhaltenen Gelfilme sind in Wasser quellbar, aber nicht löslich. Alle Filme sind in getrocknetem Zustand völlig transparent. Einige der Filme zeigen in gequollenem Zustand leichte bis mittlere Trübungen und/oder weisen eine körnige Oberfläche auf (Beisp. 4, 8, 9, 12). Bei den Beispielen 2, 4 und 6 weist die Masse vor der Filmherstellung eine sehr hohe Viskosität auf, die dazu führt, daß während der Beschichtung Unregelmäßigkeiten im Strichbild und damit Schwankungen im Flächengewicht des getrockneten Films auftreten. Die Rezepturen gemäß den Beispielen 1, 5, 7, 10, 11 und 16 sind in der Masseherstellung am besten zu handhaben, erlauben die Einarbeitung weiterer Hilfsstoffe und/oder Wirkstoffe in einem genügend weiten Rahmen und liefern sowohl im getrockneten als auch im gequollenen Zustand transparente, homogene Filme hoher Festigkeit.

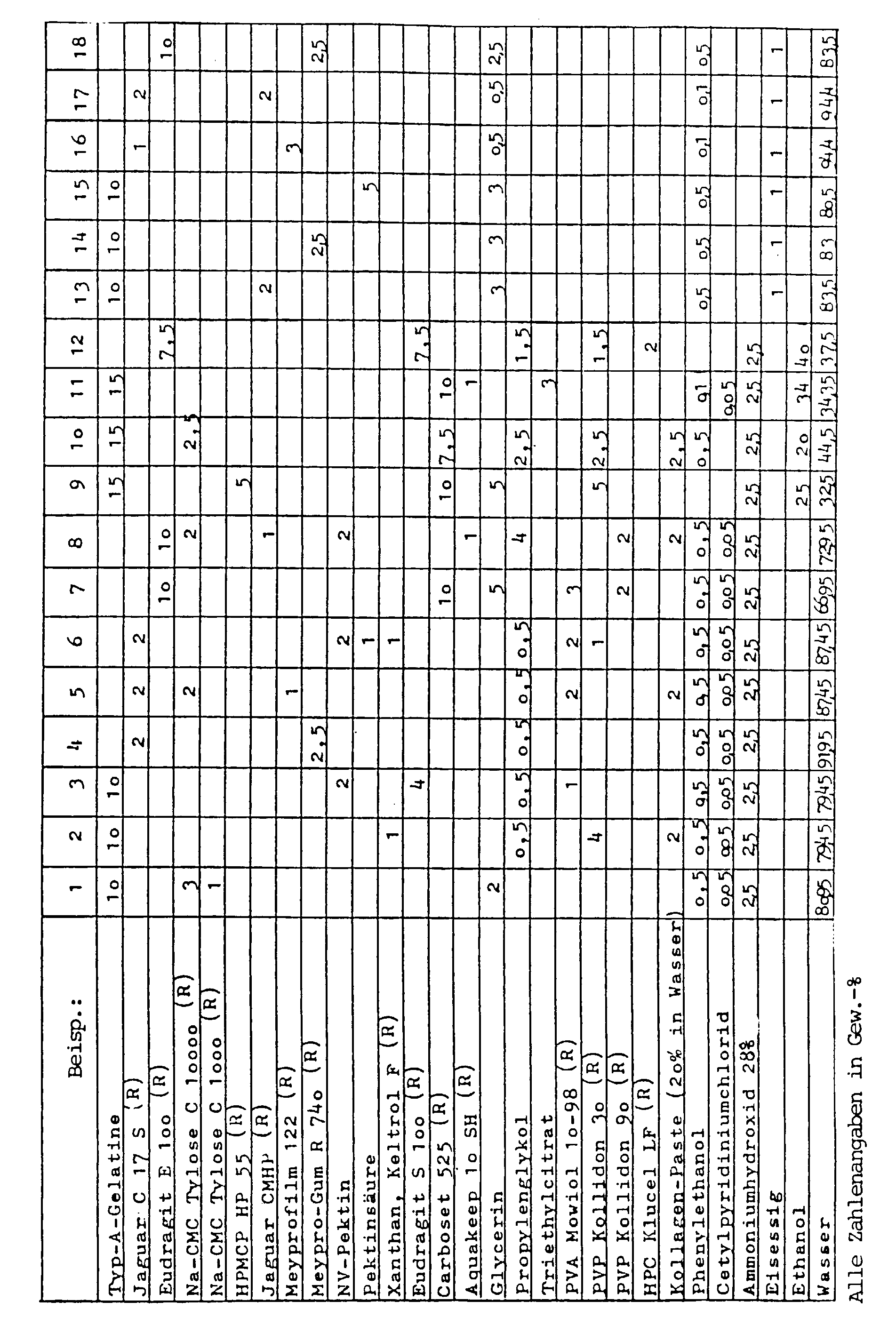

### Beispiel 19

Zu 235 ml keimarmen, deionisiertem Wasser werden 20 g Phenylethanol, 0,04 g Cetylpyridiniumchlorid und 2,5 ml Ammoniumhydroxid 28% gegeben. Die Mischung wird auf ca. 65°C erwärmt. Nacheinander werden 30,0 g Glycerin, 13,0 g Polyvinylalkohol (Mowiol 28-99^{R}), 35,0 g Polyvinylpyrrolidon (Kollidon 30^{R}), 75,0 g Typ-A-Gelatine, 15,0 g Na-carboxymethylcellulose C 10000 (Tylose^{R}), 2,5 g Na-carboxymethylcellulose C 1000 (Tylose^{R}), 35,0 g Saccharose 50,0 g medizinischer Honig und 35,0 g Pantothenylalkohol zugegeben. Im verschlossenen Gefäß wird jeweils solange gerührt und homogenisiert, bis die zugegebene Substanz klar gelöst ist. Anschließend wird die Masse auf eine Temperatur von 45°C kaltgerührt und es werden 20,0 g Kollagenpaste (20% in Wasser) eingerührt. Die Masse wird unter Vakuum luftblasenfrei gerührt und bei einer Temperatur von 45°C mit einem Flächengewicht von 2340 g/m² auf einen 40 µm-Polyurethanfilm ausgestrichen und im Trockenkanal bei 80°C auf eine Restfeuchte von ca. 25%, entsprechend einem Flächengewicht von 1680 g/m², getrocknet. Nach der Trocknung wird der Film mit einer silikonisierten Folie abgedeckt, auf Format gestanzt und konfektioniert. Der Gelfilm ist transparent, haftet auf der Haut und kann aufgrund seines hohen Flüssigkeitsaufnahmevermögens als Abdeckung für Wunden mit starker Sekretabsonderung eingesetzt werden.

### Beispiel 20

Zu 100 ml keimarmem, deionisiertem Wasser werden 0,45 g Phenylethanol, 0.01 g Cetylpyridiniumchlorid und 1,0 ml Ammoniumhydroxid 28% gegeben. Die Mischung wird auf ca. 65°C erwärmt. Nacheinander werden 7,0 g Glycerin, 10,0 g Carboset 525^{R}, 20,0 g Typ-A-Gelatine, 7,0 g Polyvinylpyrrolidon (Kollidon 30^{R}), 5,0 g Saccharose, 10,0 g medizinischer Honig und 7,0 g Pantothenylalkohol zugegeben. Im verschlossenen Gefäß wird jeweils solange gerührt und homogenisiert, bis die zugegebene Substanz klar gelöst ist. Die Masse wird auf eine Temperatur von 50°C kaltgerührt, und es werden 10,0 g Kollagenpaste (20% in Wasser) eingerührt. Die Masse wird bei einer Temperatur von 50°C mit einem Flächengewicht von 775 g/m² auf einen 40 µm-Polyurethanfilm ausgestrichen und im Trockenkanal bei 80°C auf eine Restfeuchte von ca. 25%, entsprechend einem Flächengewicht von 400 g/m², getrocknet. Nach der Trocknung wird der Film mit einer silikonisierten Folie abgedeckt, auf Format gestanzt und konfektioniert. Der Gelfilm ist transparent, haftet auf der Haut und kann z.B. als Abdeckung für Wunden mit geringer Sekretabsonderung oder für Bagatellwunden eingesetzt werden.

### Beispiel 21

Zu 34,245 ml keimarmen, deionisiertem Wasser werden 20,0 g Ethanol, 0,25 g Phenylethanol, 0,005 g Cetylpyridiniumchlorid und 1,0 ml Ammoniumhydroxid 28% gegeben. Die Mischung wird auf ca. 45°C erwärmt. Nacheinander werden klar gelöst 5,0 g Hydroxypropylcellulose (Klucel LF^{R}), 3,0 g Na-carboxymethylcellulose C 10000 (Tylose^{R}), 1,0 g Na-carboxymethylcellulose C 1000 (Tylose^{R}), 3,0 g Glycerin, 25,0 g Typ-A-Gelatine, 2,5 g Kollagenpaste (20% in Wasser), 2,5 g Myrrhentinktur und 2,5 g Salbeitinktur. Die Masse wird bei einer Temperatur von 45°C mit einem Flächengewicht von 300 g/m² auf eine silikonisierte Polyesterfolie gestrichen und im Trockenkanal bei 60°C auf eine Restfeuchte von ca. 25%, entsprechend einem Flächengewicht von 150 g/m², getrocknet. Nach der Trocknung wird der Gelfilm mit einer silikonisierten Folie abgedeckt, auf Format gestanzt und konfektioniert. Der Gelfilm wird auf die Mundschleimhaut appliziert, haftet auf dieser und setzt im Zuge der Durchfeuchtung und Quellung die Wirkstoffe frei, die der Bekämpfung von Entzündungen im Mund- und Rachenraum dienen.

### Beispiel 22

Zu 36,0 ml Ethanol und 16,0 g keimarmem, deionisiertem Wasser werden 0,5 g Phenylethanol, 1,0 ml Ammoniumhydroxid 28%, 7,0 g Eudragit E 100^{R}, 7,0 g Carboset 525^{R}, 3,5 g Propylenglykol und 5,0 Kollidon 30^{R} gegeben. Es wird gerührt und homogenisiert bis die zugegebenen Substanzen klar gelöst sind. Die Lösung wird portionsweise in eine Mischung aus 32,0 g Acronal V 205^{R}, 17,0 g Acronal 85 D^{R} und 1,0 g Ammoniumhydroxid 28% langsam eingerührt. Die Masse wird bei Raumtemperatur mit einem Flächengewicht von 375 g/m² auf einen 40 µm-Polyurethanfilm ausgestrichen und im Trockenkanal bei 60°C auf eine Restfeuchte von ca. 5%, entsprechend einem Flächengewicht von ca. 150 g/m², getrocknet. Nach der Trockung wird der Film mit einer silikonisierten Folie abgedeckt, auf Format gestanzt und konfektioniert. Der Gelfilm kann als Haftkleber für medizinische Artikel verwendet werden und hat auch auf schweißnasser Haut eine gute Haftung und gute Kohäsion.

### Beispiel 23

Zu 44,0 ml keimarmen, deionisiertem Wasser werden 0,5 g Phenylethanol und 1,0 ml Ammoniumhyroxid 28% gegeben. Die Mischung wird auf ca. 65°C erwärmt. Nacheinander werden 2,0 g Mowiol 10-98^{R}, 5,0 g Glycerin, 2,0 g Tylose C 1000^{R}, 5,0 g Carboset 525^{R} und 10,0 g Typ-A-Gelatine zugegeben. Im verschlossenen Gefäß wird jeweils solange gerührt und homogenisiert, bis die zugegebene Substanz klar gelöst ist. Die Masse wird auf eine Temperatur von 50°C kaltgerührt, und es werden 2,5 g Kollagenpaste (20% in Wasser) eingerührt. Die Masse wird bei dieser Temperatur gehalten (Wasser-Phase).

10,0 g Arnikaöl, 6,0 g Campher, 4,0 g Polyethylenglycerolmonostearat und 8,0 g Cetylstearylalkohol werden auf 50°C erwärmt und langsam bis zur klaren Lösung gerührt (Öl-Phase). In die Ölphase wird die Wasserphase bei 50°C unter Rühren und Homogenisieren portionsweise eingearbeitet. Die homogene cremefarbene Masse wird bei einer Temperatur von 50°C mit einem Flächengewicht von 600 g/m² auf eine 30 µm-Polyesterfolie ausgestrichen und im Trockenkanal bei 50°C auf einen Restwassergehalt von ca. 25%, entsprechend einem Flächengewicht von 410 g/m², getrocknet. Nach der Trocknung wird der Film mit einer silikonisierten Folie abgedeckt, auf Format gestanzt und konfektioniert (gem. Fig. 8). Der Gelfilm kann topisch zur Durchblutungsförderung und Entzündungshemmung z.B. bei leichten rheumatischen Beschwerden oder Sportverletzungen eingesetzt werden.

### Beispiel 24

Zu 41,0 ml keimarmem, deionisiertem Wasser werden 0,5 g Phenylethanol und 1,0 ml Ammoniumhydroxid 28% gegeben. Die Mischung wird auf ca. 65°C erwärmt. Nacheinander werden 2,0 g HPMCP HP 55^{R}, 3,0 g Eudragit S 100^{R}, 2,0 g Carboset 525^{R}, 2,0 g Kollidon 30^{R}, 2,0 g Propylenglykol, 5,0 g Typ-A-Gelatine und 2,5 g Kollagenpaste (20% in Wasser) zugegeben. Wenn alle Bestandteile klar gelöst sind, wird die Lösung auf ca. 45°C abgekühlt und unter langsamem Rühren portionsweise zu 40,0 g Acronal V 205^{R} gegeben (Wasser-Phase).

6,0 g Tween 20, 9,0 g Tween 61, 8,0 g Eucalyptusöl, 10,0 g Latschenkieferöl und 2,0 g Anisöl werden bei Raumtemperatur miteinander vermischt und homogenisiert (Öl-Phase).

In die Ölphase wird die warme Wasserphase unter Rühren und Homogenisieren portionsweise eingearbeitet. Die Masse wird dann mit einem Flächengewicht von ca. 680 g/m² auf eine poröse 15 µm-Polyesterfolie ausgestrichen und im Trockenkanal bei 45°C auf einen Restwassergehalt von ca. 10%, entsprechend einem Flächengewicht von 400 g/m², getrocknet. Nach der Trocknung wird der Film mit einer silikonisierten Folie abgedeckt, auf Format gestanzt und konfektioniert (gem. Fig. 12). Der Gelfilm kann bei Erkältungskrankheiten im oberen Brustbereich auf die Haut geklebt werden und setzt nach Entfernen der rückseitigen Abdeckung bei Erwärmen auf Körpertemperatur langsam die ätherischen Öle zur Inhalation frei.

Nachfolgend werden die der Verdeutlichung der Erfindung dienenden Figuren 1 bis 12 erläutert:

Fig. 1 stellt einen Gelfilm (1) dar, der auf einer Seite mit einer undurchlässigen Rückschicht (2) und auf der gegenüberliegenden Seite mit einer ablösbaren, undurchlässigen Schutzschicht (3) versehen ist.

Fig. 2 beschreibt einen mehrschichtigen Gelfilm (Laminat), in dem der Gelfilm (1) in zwei Schichten (1′, 1˝) unterteilt ist.

Fig. 3 stellt ebenfalls einen Mehrschichtverbund dar, in dem ein substanz- bzw. wirkstofffreier Gelfilm (1) sandwichartig von zwei lipophilen Polymerfilmen (4, 4′) umgeben ist, die beide substanz- bzw. wirkstoffhaltig sind. Abgedeckt wird diese Anordnung wieder durch die Rückschicht (2) und Schutzschicht (3).

In Fig. 4 ist ein substanz- bzw. wirkstofffreier Gelfilm (1) dargestellt, der an einer Seite mit der Schutzschicht (3) bedeckt ist und der als Steuermembran für einen substanz- bzw. wirkstoffhaltigen Polymerfilm (4), der mit der Rückschicht (2) abgedeckt ist, dient.

In Fig. 5 ist der wirkstoffhaltige Gelfilm (1) auf der dem Substrat zugewandten Seite mit einem wirkstofffreien, lipophilen Polymerfilm (4), der als Steuermembran dient, kombiniert.

Fig. 6a und 6b zeigen eine Ausführungsform des Gelfilms (1), in dem dieser zumindest teilweise von lipophilem Polymerfilm (4) umgeben ist, oder dieser Film selbst zumindestens teilweise ein lipophiles Element umgibt. Beide Elemente können wirkstoffhaltig sein.

Fig. 7a zeigt eine weitere Ausführungsform, in der der Gelfilm (1), der als Steuerelement wirkt, einen lipophilen Wirkstoffspeicher (4) umschließt; in Fig. 7b ist der als Speicherelement wirkende Gelfilm (1) in einem lipophilen, als Steuerelement wirkenden Film (4) vollständig eingebettet. Lipophiles Speicherelement (4) bzw. Gelfilm (1) als Speicher können auch mit einer Oberfläche direkt an die Rückschicht (2) angrenzen (Fig. 7c und 7d).

Fig. 8a und 8b zeigen Vorrichtungen, bei denen ein Gelfilm (1) mit einer Schutzschicht (3) und mit einer Rückschicht (2), die größere Flächenabmessungen als der Gelfilm (1) aufweist und die ganz (Fig. 8a) oder teilweise (Fig. 8b) mit einem Klebstoffilm (5) beschichtet ist, bedeckt ist.

Fig. 9, 10a und 10b zeigen Ausführungsformen eines Gelfilms (1) mit Klebstoffschicht (5), wobei diese Klebstoffschicht diskontinuierlich, d.h. unterbrochen, den gesamten Gelfilm (Fig. 10a) oder Teile des Gelfilms (Fig. 10b) bedeckt.

In Fig. 11a und 11b ist ein Gelfilm (1) dargestellt, der mit einem Steuerelement (6) kombiniert ist, das eine durchbrochene Schicht darstellt und über eine Klebstoffschicht (5) mit der Rückschicht (2) verbunden ist (Fig. 11a). In Fig. 11b befindet sich zwischen Gelfilm (1) und Schutzschicht (3) eine zweite Klebstoffschicht (5′), die nach Entfernen der Schutzschicht (3) zur Verankerung des Gelfilms (1) auf einer beliebigen Oberfläche dient.

In Fig. 12 ist eine Vorrichtung dargestellt, bei der der Gelfilm (1) auf der Rückseite mit einer porösen Steuermembran (6) abgedeckt ist, die größere Flächenabmessungen als der Gelfilm (1) aufweist und deren überstehende Teile mit einer Klebstoffschicht (5′) bedeckt sind. Die poröse Fläche der Steuermembran (6) ist mit einer Rückschicht (2), die mit einem Klebstoffilm (5) beschichtet ist, abgedeckt. Die Rückschicht (2) hat größere Flächenabmessungen als die poröse Fläche der Steuermembran (6), z.B. in Form einer überstehenden Lasche, so daß die Rückschicht (2) mit dem Klebstoffilm (5) vor der Anwendung leicht entfernt werden kann.

## Patentansprüche

1. Verfahren zur Herstellung eines flexiblen, hydrophilen, in Wasser quellbaren, nicht löslichen Gelfilmes, enthaltend
- 0,5 bis 30 Gew.-% mindestens eines wasserlöslichen bei neutralem pH anionaktiven Polymers,
- 0,5 bis 50 Gew.-% mindestens eines wasserlöslichen bei neutralem pH kationaktiven Polymers,
- 0,1 bis 20 Gew.-% mindestens eines Feuchthaltemittels,
- 0,1 bis 70 Gew.-% Wasser bzw. wässrige Lösemittel,
- 0 bis 75 Gew.-% wasserlösliche oder wasserdispergierbare Hilfsstoffe,
- 0 bis 50 Gew.-% Wirkstoff, mit den folgenden Arbeitsschritten :
a) in einem ersten Arbeitsschritt wird dem Lösemittel, welches Wasser oder eine Mischung von Wasser mit darin mischbaren Lösemittel enthält, zuerst ein die Reaktion der Polymeren unterschiedlicher Ladung durch reversible Salzbildung verhindernder flüchtiger Zusatz basischen oder sauren Charakters, der eine Siedetemperatur zwischen 25° und 120°, vorzugsweise zwischen 40° und 80° aufweist, zugegeben, wonach
b) in einem zweiten Arbeitsschritt in der nach a) erhaltenen Lösung wasserlösliches, bei neutralem pH anionaktives Polymer, wasserlösliches bei neutralem pH kationaktives Polymer, Feuchthaltemittel und gegebenenfalls weitere Rezepturbestandteile gelöst, emulgiert oder suspendiert werden, und die Temperatur der Lösung während des Lösungsvorganges die Siedetemperatur des flüchtigen Zusatzes nicht übersteigt, wobei
als flüchtige Zusätze mit saurem Charakter Chlorwasserstoffsäure, Kohlensäure, Essigsäure oder Ameisensäure und
als flüchtige Zusätze mit basischem Charakter Ammoniumhydroxid oder primäre, sekundäre oder tertiäre Amine mit Siedepunkten zwischen 25°C und 120°C, vorzugsweise Isopropylamin, Tertiärbutylamin, Diethylamin, Isobutylamin, Diisopropylamin, Isoamylamin oder Triethylamin verwendet werden, und daß
als wasserlösliches anionaktives Polymer mindestens ein Polymer aus der Gruppe umfassend anionaktive Cellulosederivate, anionaktive Stärkederivate, anionaktive Galaktomannanderivate, anionaktive pflanzliche Polysaccharide, anionaktive Polyacrylate und Polymethacrylate, sowie
als kationaktives Polymer mindestens ein Polymer aus der Gruppe umfassend kationaktive Typ-A-Gelatine, kationaktive Galaktomannanderivate, kationaktive Polyacrylate und Polymethacrylate zugesetzt werden, und daß
die so erhaltene Mischung in eine Form gegossen oder auf einen Träger gestrichen und die ausgegossene oder ausgestrichene Mischung auf eine Restfeuchte zwischen maximal 25% und minimal 5% getrocknet und dabei auch der flüchtige Zusatz entfernt wird.

2. Verfahren nach Anspruch 1, dadurch gekennzeichnet, daß als Feuchthaltemittel Alkohol , vorzugsweise Glycerin, Propylenglykol oder Sorbit verwendet wird.

3. Verfahren nach Anspruch 1, dadurch gekennzeichnet, daß als wasserlösliche oder wasserdispergierbare Hilfsstoffe Weichmacher, Verdickungsmittel, Penetrationsbeschleuniger , Klebrigmacher, Konservierungsmittel, Desinfektionsmittel, pH-Regulatoren, Antioxidantien, Emulsionsstabilisatoren, Vernetzungsmittel, Füllstoffe und/oder Schaumstabilisierungsmittel verwendet werden.

4. Verfahren nach Anspruch 3, dadurch gekennzeichnet, daß als Verdickungsmittel Kollagen verwendet wird.

5. Verfahren nach Anspruch 3, dadurch gekennzeichnet, daß als Klebrigmacher natürliche, semisynthetische und/oder synthetische Harze oder Gummen verwendet werden.

6. Verfahren nach Anspruch 1, dadurch gekennzeichnet, daß als Klebrigmacher Honig, Zucker und/oder Pantothenylalkohol verwendet werden.

7. Verfahren nach einem der Ansprüche 1 bis 6, dadurch gekennzeichnet, daß der Gelfilm eine lipophile innere Phase enthält.

8. Verfahren nach einem der Ansprüche 1 bis 6, dadurch gekennzeichnet, daß der Gelfilm transparent hergestellt ist.

9. Verfahren nach einem der Ansprüche 1 bis 8, dadurch gekennzeichnet, daß der Gelfilm mit einer porösen Struktur hergestellt ist.

10. Verfahren nach einem der Ansprüche 1 bis 9, dadurch gekennzeichnet, daß der Gelfilm mehrschichtig aufgebaut ist.

11. Verfahren nach einem der Ansprüche 1 bis 10, dadurch gekennzeichnet, daß der Gelfilm biologisch abbaubar ist.

12. Verwendung des flexiblen, hydrophilen Gelfilms nach einem der Ansprüche 1 bis 11, zur Erzeugung von Wechselwirkungen zwischen dem Gelfilm und einem festen, flüssigen oder gasförmigen Substrat.

13. Verwendung nach Anspruch 12, dadurch gekennzeichnet, daß das Substrat intakte oder geschädigte Haut ist.

14. Verwendung nach Anspruch 12, dadurch gekennzeichnet, daß das Substrat Schleimhaut ist.

15. Verwendung nach Anspruch 12, dadurch gekennzeichnet, daß das Substrat pflanzlichen Ursprungs ist.

16. Verwendung des flexiblen, hydrophilen Gelfilms nach einem der Ansprüche 12 bis 15, dadurch gekennzeichnet, daß der Gelfilm in auf dem Substrat fixierbare Abschnitte zerteilt wird, die mit einer die Austrocknung verhindernden undurchlässigen Rückschicht und auf der gegenüberliegenden Seite mit einer ablösbaren, undurchlässigen Schutzschicht versehen sind.

## Claims

1. A process for the production of a flexible, hydrophilic, water-swellable, insoluble gel film containing
- 0.5 to 30%-wt of at least one water-soluble polymer being anion-active at neutral pH
- 0.5 to 50%-wt of at least one water-soluble polymer being cation-active at neutral pH
- 0.1 to 20%-wt of at least one moisturizer
- 0.1 to 70%-wt of water or aqueous solvents, respectively
- 0 to 75%-wt of water-soluble or water-dispersible auxiliaries
- 0 to 50%-wt of active substance, the process comprising the following steps:
(a) in a first step, a volatile additive of basic or acidic character preventing the reaction of polymers of different charge by reversible salt formation and having a boiling temperature between 25°C and 120°C, preferably between 40°C and 80°C is added to the solvent, which contains water or a mixture of water and solvents which are miscible with water,
(b) in a second step, in the solution obtained according to (a) there are dissolved, emulsified or suspended a water-soluble polymer being anion-active at neutral pH, a water-soluble polymer being cation-active at neutral pH, moisturizer(s) and optionally further formulation components, the temperature of the solution during the dissolving step not exceeding the boiling temperature of the volatile additive, whereby
as volatile additives having acidic character there are used hydrochloric acid, carbonic acid, acetic acid or formic acid, and as volatile additives having basic character there are used ammonium hydroxide or primary, secondary and tertiary amines having boiling points between 25°C and 120°C, preferably isopropylamine, tertiary butylamine, diethylamine, isobutylamine, diisopropylamine, isoamylamine or triethylamine, and that
as water-soluble anion-active polymer at least one polymer of the group comprising anion-active cellulose derivatives, anion-active starch derivatives, anion-active galactomannan derivatives, anion-active vegetable polysaccharides, anion-active polyacrylates and polymethacrylates, and
as cation-active polymer at least one polymer of the group comprising cation-active type-A-gelatin, cation-active galactomannan derivatives, cation-active polyacrylates and polymethacrylates are added, and that
the resultant mixture is cast into a mold or spread onto a carrier, and the cast or spread mixture is dried to a residual moisture of between 25% at maximum and 5% at minimum, thereby also removing the volatile additive.

2. The process according to claim 1, characterized in that as moisturizer alcohol, preferably glycerol, propylene glycol, or sorbitol is used.

3. The process according to claim 1, characterized in that as water-soluble or water-dispersible auxiliaries, softeners, thickeners, penetration accelerators, tackifiers, preserving agents, disinfectants, pH-regulators, antioxidants, emulsion stabilizers, cross-linking agents, fillers and/or foam stabilizers are used.

4. The process according to claim 3, characterized in that collagen is used a thickening agent.

5. The process according to claim 3, characterized in that as tackifiers natural, semi-synthetic and/or synthetic resins or gums are used.

6. The process according to claim 1, characterized in that as tackifiers honey, sugar and/or panthenol are used.

7. The process according to any one of claims 1 - 6, characterized in that the gel film contains a lipophilic inner phase.

8. The process according to any one of claims 1 - 6, characterized in that the gel film is produced such that it is transparent.

9. The process according to any one of claims 1 - 8, characterized in that the gel film is produced such that it has a porous structure.

10. The process according to any one of claims 1 - 9, characterized in that the gel film has a multi-layered structure.

11. The process according to any one of claims 1 - 10, characterized in that the gel film is biologically decomposable.

12. The use of the flexible, hydrophilic gel film according to any one of claims 1 to 11 for the creation of interactions between the gel film and a solid, liquid or gaseous substrate.

13. The use according to claim 12, characterized in that the substrate is intact or damaged skin.

14. The use according to claim 12, characterized in that the substrate is mucous membrane.

15. The use according to claim 12, characterized in that the substrate is of vegetable origin.

16. The use of the flexible, hydrophilic gel film according to any one of claim 12 - 15, characterized in that the gel film is divided into sections which can be secured on the substrate and which are provided with an impermeable backing layer preventing drying out, and on the opposite side with a removable, impermeable protective layer.

## Revendications

1. Procédé de préparation d'un film de gel souple, hydrophile, insoluble dans l'eau, gonflable à l'eau, contenant
- 0,5 à 30% en poids d'au moins un polymère soluble dans l'eau, anioniquement actif à pH neutre,
- 0,5 à 30% en poids d'au moins un polymère soluble dans l'eau, cationiquement actif à pH neutre,
- 0,1 à 20% en poids d'au moins un agent de rétention d'eau,
- 0,1 à 70% en poids d'eau ou des solvants aqueux,
- 0 à 75% en poids d'adjuvants solubles dans l'eau ou dispersibles dans l'eau,
- 0 à 50% en poids de principe actif,
comportant les étapes opératoires suivantes :
a) au cours d'une première étape opératoire, on ajoute au solvant, qui contient de l'eau ou un mélange d'eau et de solvants miscibles à l'eau, d'abord un additif de caractère basique ou acide, volatil, inhibant la réaction des polymères de charge différente par formation de sel réversible, qui présente une température d'ébullition comprise entre 25 et 120°C, de préférence, entre 40 et 80°C, ensuite,
b) au cours d'une seconde étape opératoire, au cours de laquelle la solution obtenue selon a), le polymère soluble dans l'eau anioniquement actif à pH neutre, le polymère soluble dans l'eau, cationiquement actif à pH neutre, des agents de rétention d'eau et éventuellement d'autres constituants de la composition sont dissous, émulsionnés ou mis en suspension et la température de la solution au cours du processus de dissolution ne dépasse pas la température d'ébullition de l'additif volatil,
où
on utilise, à titre d'additifs volatils à caractère acide, l'acide chlorhydrique, l'acide carbonique, l'acide acétique, ou l'acide formique et, à titre d'additifs volatils à caractère basique, de l'hydroxyde d'ammonium, ou des amines primaires, secondaires, ou tertiaires, possédant des points d'ébullition compris entre 25°C et 120°C, de préférence, l'isopropylamine, la tert-butylamine, le diéthylamine, l'isobutylamine, la diisopropylamine, l'isoamylamine, ou la triéthylamine
et on ajoute
à titre de polymère anioniquement actif, soluble dans l'eau, au moins un polymère appartenant au groupe qui comprend des dérivés de la cellulose anioniquement actifs, des dérivés de l'amidon anioniquement actifs, des dérivés du galactomannane anioniquement actifs, des polysaccharides végétaux anioniquement actifs, des polyméthacrylates et des polyacrylates, anioniquement actifs,
ainsi que
à titre de polymère cationiquement actif, au moins un polymère appartenant au groupe comprenant des gélatines du type A cationiquement actives, des dérivés du galactomannane cationiquement actifs, des polyméthacrylates et des polyacrylates, cationiquement actifs,
et
on coule le mélange ainsi obtenu dans une forme, ou on l'étale sur un support et on sèche le mélange coulé ou étalé jusqu'à une humidité résiduelle comprise entre au maximum 25% et au minimum 5% et on élimine ainsi aussi l'additif volatil.

2. Procédé suivant la revendication 1, caractérisé en ce qu'à titre d'agents de rétention d'eau, on utilise de l'alcool, de préférence la glycérine, le propylèneglycol, ou la sorbite.

3. Procédé suivant la revendication 1, caractérisé en ce que, à titre d'adjuvants solubles dans l'eau ou dispersibles dans l'eau, on utilise des plastifiants, des épaississants, des accélérateurs de pénétration, des agents conférant de l'adhésivité, des conservateurs, des agents désinfectants, des régulateurs du pH, des antioxydants, des stabilisateurs d'émulsion, des agents de réticulation, des charges et/ou des agents de stabilisation de la mousse.

4. Procédé suivant la revendication 3, caractérisé en ce que l'on utilise le collagène à titre d'épaississant.

5. Procédé suivant la revendication 3, caractérisé en ce qu'à titre d'agents conférant de l'adhésivité, on utilise des résines ou gommes naturelles, semi-synthétiques et/ou synthétiques.

6. Procédé suivant la revendication 1, caractérisé en ce qu'à titre d'agent conférant de l'adhésivité, on utilise du miel, du sucre et/ou l'alcool pantothénylique.

7. Procédé suivant l'une quelconque des revendications 1 à 6, caractérisé en ce que le film de gel contient une phase interne lipophile.

8. Procédé suivant l'une quelconque des revendications 1 à 6, caractérisé en ce que l'on fabrique le film de gel de manière à ce qu'il soit transparent.

9. Procédé suivant l'une quelconque des revendications 1 à 8, caractérisé en ce que l'on fabrique le film de gel de manière à ce qu'il comporte une structure poreuse.

10. Procédé suivant l'une quelconque des revendications 1 à 9, caractérisé en ce que le film de gel est de constitution multicouche.

11. Procédé suivant l'une quelconque des revendications 1 à 10, caractérisé en ce que le film de gel est biologiquement dégradable.

12. Utilisation du film de gel souple et hydrophile suivant l'une quelconque des revendications 1 à 11, pour l'obtention d'activités d'échange entre le film de gel et un substrat solide, liquide, ou gazeux.

13. Utilisation suivant la revendication 12, caractérisée en ce que le substrat est de la peau intacte ou endommagée.

14. Utilisation suivant la revendication 12, caractérisée en ce que le substrat est une muqueuse.

15. Utilisation suivant la revendication 12, caractérisée en ce que le substrat est d'origine végétale.

16. Utilisation du film de gel souple et hydrophile suivant l'une quelconque des revendications 12 à 15, caractérisée en ce que l'on subdivise le film de gel en découpes pouvant se fixer sur le substrat, lesquelles découpes sont pourvues d'une couche dorsale imperméable empêchant le dessèchement et, sur le côté opposé, d'une couche protectrice imperméable et amovible.
